# EUROPEAN PATENT APPLICATION

(11) **EP 4 497 386 A1**
(43) Date of publication of application: **29.01.2025**
(21) Application number: 23306311.4
(22) Date of filing: 28.07.2023
(51) Int. Cl.: A61B 5/346

(54) **METHOD FOR DETECTING RISK OF TORSADES DE POINTES IN LONG QT PATIENTS**

(71) Applicant: Assistance Publique - Hôpitaux de Paris, 75012 Paris (FR); Université Paris Cité, 75006 Paris (FR); Sorbonne Université, 75006 Paris (FR); Institut de Recherche pour le Développement, 13002 Marseille (FR); Institut National de la Santé et de la Recherche Médicale (INSERM), 75013 Paris (FR)
(72) Inventor: EXTRAMIANA, Fabrice, 94100 SAINT MAUR DES FOSSÉS (FR); PRIFTI, Edi, 92160 ANTONY (FR); FALL, Ahmad, 93141 BONDY (FR); SALEM, Joe-Elie, 75015 PARIS (FR); DENJOY, Isabelle, 75011 PARIS (FR); ZUCKER, Jean-Daniel, 75013 PARIS (FR); LEENHARDT, Antoine, 75017 PARIS (FR)
(74) Representative: Flesselles, Bruno F.G.

(57) **Abstract**

The invention relates to the detection of the risk for a patient with a congenital Long QT (LQT) condition for having a torsades-de-pointes event, and the mechanisms underlying such risk, in particular via the use of neural networks.

## Description

The invention relates to the detection of the risk for a patient with a congenital Long QT (LQT) condition for having a torsade de pointes event, and the mechanisms underlying such risk, in particular via the use of neural networks.

Torsades de Pointes (TdP) is an arrhythmia that can deteriorate to ventricular fibrillation and sudden death. It occurs essentially in the congenital long QT syndromes or as a rare side effect of QT-prolonging cardiac and non-cardiac drugs.

The current detection strategy is based on the QT measurements (i.e., interval between the beginning of the QRS complex and the end of the T wave, traditionally, using generally the lead II of the electrocardiogram (ECG)). TdP risk is a major issue for the drug industry, and can be the reason for withdrawing drugs from market.

Another method was described in US 20190059764, which discloses an algorithm using the QT interval corrected with respect to the heart rate, the maximum amplitude of the first peak of the T wave and the interval between the maximum first peak of the T wave and the end of the T wave. Although efficient, this algorithm may not always be easy to implement, as it requires comparing the ECG before and after intake of a QT prolonging drug.

WO 2020/245322 and Prifti et al (Eur Heart J. 2021 Oct 7;42(38):3948-3961. doi: 10.1093/eurheartj/ehab588) disclose systems and methods for deep learning analysis of electrocardiogram for risk prediction of drug-induced arrhythmias and diagnosis of long QT syndrome.

Congenital long QT syndrome (LQTS) is a rare hereditary disease (prevalence = 1/5000 to 1/2000) that associates a prolongation of the QT interval on the ECG and the potential occurrence of syncope and/or sudden death by ventricular arrhythmia of the type of torsades-de-pointes spontaneously stopping or degenerating into ventricular fibrillation. Advances in molecular biology have revealed the involvement of numerous genes coding for membrane ion channels or for proteins that interfere with these channels. However, the first 3 forms described (LQT1, LQT2 and LQT3) correspond to at least 75% of cases. The interpretation of the significance of the variants found in the other genes is increasingly discussed.

The main current challenge in congenital long QT syndrome remains the prevention of sudden death. This risk has been drastically reduced by use of beta-blockers. In particular, the use of Nadolol was associated with a decrease of more than 60% (HR=0.38) in potentially lethal arrhythmic events. However, the efficacy of beta-blockers appears to be excellent in LQT1 but less satisfactory in LQT2 and 3, and residual mortality persists despite beta-blocker therapy. This premature mortality could be prevented by implantation of an automatic implantable defibrillator (ICD). However, this technique is associated with very high complication rates that are difficult to accept in primary prevention in this young population. One can also perform left cardiac sympathetic denervation (LCSD), but that would also require surgical procedure.

Improving the management of LQTS therefore requires improved risk stratification for sudden death (i.e., identification of patients that present a higher risk of having a sudden death than the average risk in the population of patients, herein designated as an increased risk). This stratification is currently based on clinical data including presenting symptoms, genetic type of LQTS (LQT1, 2, or 3), and QTc interval duration. Therefore, it is desired to stratify the population of patients, in at least two groups, one group of patients having a below than average risk of having sudden death or a TdP event, and one risk of patients having a higher-than-average risk of having these events, the average risk being the occurrence of these events in the whole population of patients (i.e. patients with congenital LQTS).

Indeed, among the patient with congenital LQTS, it is postulated that the electrophysiological abnormalities that serve as a substrate for arrhythmias are constantly present on the ECG (due to the hereditary and therefore congenital nature of the initial abnormality causing the syndrome). Such abnormalities are thus present before occurrence of a Life Threatening Adverse Event (LTAE, an event the occurrence of which places the patient or subject at immediate risk of death, including aborted cardiac arrest (requiring external defibrillation as part of the resuscitation or internal defibrillation), LQTS-related Sudden Cardiac Death, irregular heartbeat or Torsades-de-Pointes event; it is reminded that TdP may degenerate into ventricular fibrillation leading to cardiac arrest and causing sudden cardiac death (SCD) if not aborted by resuscitation maneuvers), but will remain present after such event occurred and the patient has recovered. It is indeed observed that patients who already had a LTAE and recovered are at higher risk to have another one in the future.

Consequently, the inventors have studied that association between "event" and "ECG parameters" for patients having had a LTAE as it is the same for events occurring before diagnosis (and therefore known at diagnosis) and those occurring during follow-up. The model herein disclosed can thus be used to detect whether a patient had a LTAE or whether such patient is at higher risk of LTAE. Thus, the methods herein disclosed make it possible to "quantify" from the electrocardiogram how the genetic defect (present for the patient on which the methods are applied) has an impact on the actual arrhythmic risk (and hence the LTAE).

Mazzanti et al (J Am Coll Cardiol. 2018 Apr 17;71(15):1663-1671. doi:10.1016/j.jacc.2018.01.078) investigated the relationship between QTc duration and risk of events with the use of linear and cubic spline models. They were able to calculate the 5-year risk of LAEs (life-threatening arrhythmic events) of patients who were off therapy using a multivariable Cox model with QTc and genotype considered as independent factors. The linear model performed best. The estimated risk of LAEs increased by 15% for every 10-ms increment of QTc duration for all genotypes. Intergenotype comparison showed that the risk for patients with LQT2 and LQT3 increased by 130% and 157% at any QTc duration versus patients with LQT1. Analysis of response to beta-blockers showed that nadolol reduced the arrhythmic risk in all genotypes significantly compared with no therapy (hazard ratio: 0.38; 95% confidence interval: 0.15 to 0.93; p = 0.03).

However, these approaches are unspecific and nonintegrative of the total information contained in the electrocardiogram.

Pathophysiologically, the duration of ventricular repolarization is a poor marker. Torsades-de-pointes can occur despite a normal QT interval because it is the spatial heterogeneity of the ventricular refractory periods (determined by the local duration of ventricular repolarization) that constitutes the arrhythmogenic substrate at the origin of Torsades-de-Pointes under conditions of prolongation and/or shortening of repolarization duration. Thoracic mapping of epicardial ventricular repolarization has shown an increase in its heterogeneity in LQTS. Numerous teams and works have attempted to identify "conventional" ECG 12-derivation markers of ventricular repolarization heterogeneities in different pathologies including LQTS.

Using Machine Learning classifiers, and notably Convolutional Neural Network (CNN), the inventors showed that it is possible to identify, among patients with cLQTS type 1, 2, or 3, those with rhythmic events (syncopes or severe rhythmic events such as sudden death, recovered cardiac arrest and documented ventricular fibrillation).

The AUROC (Aurea Under Receiving Operating Curve) was 0.95 (95% Cl 0.93; 0.98) for the training set and 0.90 (95% Cl 0.84; 0.96) for the holdout set.

In a first embodiment, the invention relates to a method for determining whether a patient with a congenital long QT has an increased risk of having a Life Threatening Adverse Event (LTAE) in particular a torsades-de-pointes event or a related event thereof (i.e., syncope, aborted cardiac arrest, sudden cardiac death), comprising:
a. Receiving signal data representing a time segment of an ECG waveform of a subject patient
b. analyzing, by the processing device via a configured artificial machine learning classifier, said signal data to generate likelihoods as output of the machine learning,
wherein a likelihood higher than 0.5 indicates an increased risk for the patient to have a torsades-de-pointes related event.

In another embodiment, the invention relates to a method for detecting whether a given subject with a congenital long QT condition has an increased risk to present LTAE, in particular an episode of torsade de pointes, comprising the steps of:
a. Obtaining ECG data from the patient;
b. applying the ECG data to a machine learning classifier configured to detect patterns in the ECG data indicative of a risk for the patient to have a LTAE, in particular a torsades de pointe event,
c. obtaining an output from the machine learning classifier, wherein the output provides a likelihood of the risk for the patient to have a LTAE, in particular a torsades-de-pointes event.

In the two above methods, the output is a number, which corresponds to a class "at higher risk of TdP event (or of LTAE)" or "at lower risk of TdP event (or of LTAE)". By construction of the machine learning classifier, and by convention, it can be decided that an output higher than 0.5 corresponds to the class "at higher risk", where an output lower than 0.5 corresponds to the class "at lower risk", as in the present application.

In the context of the present application, the increased risk is determined as compared with the risk of a population of patients having congenital Long QT. As an illustration, if the general risk of the population of congenital Long QT patients to have a TdP event is x %, the risk of congenital Long QT patients as detected by the methods herein disclosed would be higher than x %. A risk of x % within a population indicates that, in the population, x % of the patients present the required phenotype (here, TdP related event). For the population of patients defined with a higher risk of having a TdP related event according to the methods herein disclosed, more than x% of the patients in the population will present this phenotype (TdP related event). Consequently, it is said that patients have an increased risk of having the phenotype.

The above methods, as well as the other methods herein disclosed are performed ex *vivo,* and are computer-implemented.

In some embodiments, the methods provide an estimation of the risk of a LTAE (or a TdP) event within a given period of time. The given period of time may be within 5 years (in the following 5 years) for patients with regular follow-up (patients that are not a *priori* at risk other than the one associated with the congenital LQTS). For patients with congenital LQTS, which are in a specific (generally transient) situation increasing the risk (such as intake of medication, fever, hypokalemia due to diarrhea/vomiting, postpartum period in LQT2, intensive sport practice (such as swimming) in LQT1...), the method can be used to identify a risk associated with this specific situation. The risk determined by the methods herein disclosed may thus occur shortly from the start of the specific situation (generally within 2 months). The method can also be used to detect changes in the ECG of the patients, and thus monitor the increase of the risk overtime during the specific risk situation. Indeed, when the risk situation persists, the LTAE risk may increase for the patient, and this can be determined by the methods herein disclosed.

In specific embodiments and as disclosed below, the signal data or ECG data is sent to a remote machine learning classifier (remote with regards to the location where the ECG is obtained) and the output (*i.e*., the increased risk or no increased risk) is sent to the patient and/or to a physician. The patient is thus assigned to one class (increased risk) or another class (no increased risk). Generally, the ECG data that is sent to the classifier would consist of the signal/data obtained during an acquisition time of at least 5 seconds, more preferably at least about 10 seconds or 20 seconds but there is no need to use data for more than 30 seconds. Data obtained from an acquisition time of 10 seconds is relevant.

As such, the output of the system that is trained relies on a classification technique, which provides a prediction, probability, or likelihood, rather than a certainty, that the patient has a risk to show a LTAE.

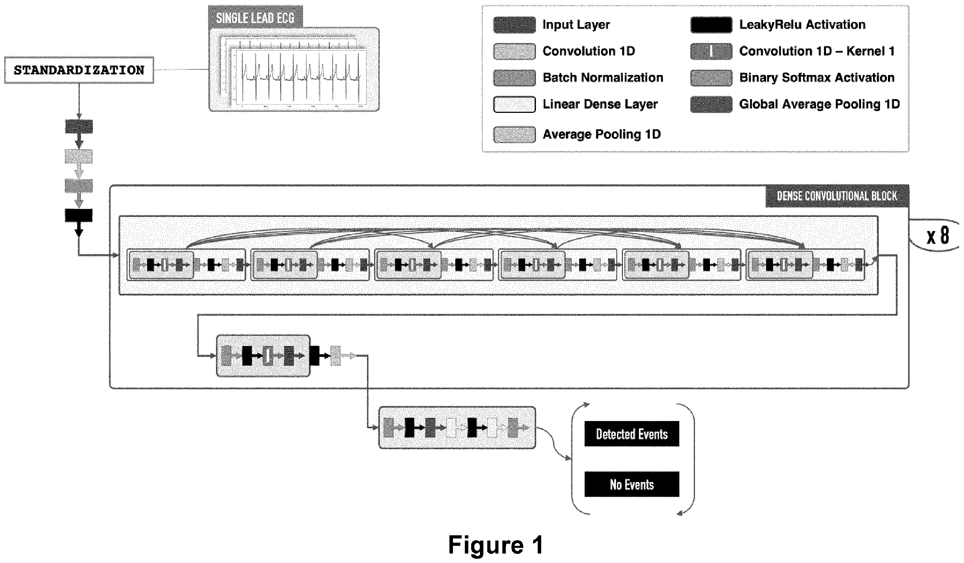

Various machine learning classifiers using one or more of the following techniques may be used to determine the likelihood of such risk, such as linear discriminant analysis, neural network, clustering techniques, support vector machine, logistic regression, naive Bayes, random forest, decision tree, and the like. It is preferred to use a Deep Neural Network, in particular a Convolutional Neural Network as the machine learning classifier for implementing the methods and in the systems herein disclosed. In a particular embodiment, a DenseNet Convolutional Neural Network can be used, *i.e*., a convolutional neural network that utilises dense connections between layers, through Dense Blocks, modules that connect all layers (with matching feature-map sizes) directly with each other such as the one disclosed in the examples or on Figure 1. In these types of neural networks, and to preserve the feed-forward nature, each layer obtains additional inputs from all preceding layers and passes on its own feature-maps to all subsequent layers. Such neural networks are particularly adapted for performing the methods herein disclosed as it allows saving of computer resources (less RAM, less GPU memory, less GPU computing power, faster computing time), which is of importance when one wishes to implement the methods at the point-of-care. In particular, it forces the network to learn with fewer things and be more robust.

When an appropriate threshold is provided, an output above the threshold indicates an increased risk of making the LTAE, whereas an output below the threshold indicates that the patient has no increased risk of making the LTAE. It is also to be noted that the method is semi-quantitative and that the higher the output the higher the risk of presenting the LTAE.

In order to increase the quality of the assessment of the risk for a given patient to have LTAE, it is possible to repeat the analysis on various ECG samples from the same patient. The ECG data that is sent to the machine learning classifier and used to calculate the output can be ECG data obtained at different times of one day, or ECG data obtained from different days, or different ECG samples from the same ECG recording.

The invention thus relates to the method described above, wherein the patient is assigned to a class of risk of having a LTAE, in particular a torsades-de-pointes event (the classes increased risk / no increased risk), in particular within the given period of time by
a. repeating the methods with different ECG data from the same patient and
b. assigning the patient in the class (increased risk / no increased risk) for which the majority of the outputs or the mean of the outputs is assigned.

Using the class of the majority of the outputs, or of the mean of the outputs increases the probability that the patient is indeed to be assigned to this class.

Repeating the classification of ECG of the patient multiple times is a preferred embodiment of the performance of the methods herein disclosed. In fact, it is advisable to repeat the method with ECG data from ECG acquired during a period of time of at least 12 hours, preferably at least 18 hours, preferably 24 hours, or even during 36 hours or 48 hours. This ECG is preferably continuously acquired and recorded during this period of time. This makes it possible to explore a larger spectrum of autonomic nervous system states as there may be some differences in such system during the day and the night, thus having an influence on the patient's heartbeat and arrhythmic risk. Such covering of a long acquisition time makes it possible to test multiple ECG of the patients (it is reminded that, in preferred embodiments, ECG data sent to the classifier is about 10 seconds long. Using an ECG acquired during 24 hours makes it possible to obtain 8640 10-sec ECG data that can be applied to the classifier (leading to 8640 outputs), or even more with overlapping segments (sliding window), thus exploring variation of the risk depending on the time of the day. In this embodiment, the patient is eventually clinically classified as at risk when the majority of the outputs, or the majority of the outputs within a given window of the day is in the "increased risk" class. Alternatively, the patient can be classified at risk, in this embodiment, at least one, more preferably at least two, more preferably at least three, more preferably at least five, most preferably at least 10 outputs are above a predetermined (high) threshold value. Such threshold value that would be of scrutiny is preferably 0.90, more preferably 0.92, more preferably 0.95, more preferably 0.97, or even 0.98 or 0.99. Such high output, especially if it is repeated (more than 2 times) notably for consecutive ECG indicates that the patient has a signal recognized, by the classifier, as very associated with LTAE (TdP) event, thus confirming that the patient is at high risk.

It is reminded that the methods herein disclosed can be used to assist the physician in the follow-up of the patients with congenital Long QT syndromes. These patients are generally regularly monitored in hospital, and it is easy to perform the methods herein disclosed on a regular basis to study the evolution of the risk. Furthermore, it is possible to install Holter monitors (small, wearable device that records the heart's rhythm) to obtain ECG data for long periods of time (12 or 24 hours) as indicated above, and especially allowing the patient to perform daily routines, thus providing information in real life (by contrast to the information obtained when a single ECG is acquired at the hospital for a short period of time). When observing evolution of the classification of the ECG data for such long time, as indicated above, the physician can use this information (as can also be used other clinical information) to adjust the treatment as described below.

The invention also relates to a method for producing a machine learning classifier (algorithm or system) capable of predicting or estimating the risk for a patient with congenital Long QT syndrome to have a LTAE, notably a torsades-de-pointes event, , comprising:
a. storing in an electronic database patient data comprising
   i. first parameters that are ECG data from various patients,
   ii. second parameters relating to the occurrence (or the non-occurrence) of a LTAE, notably a torsades-de-pointes event for each patient;
b. providing a machine learning system; and
c. training the machine learning system using the first and second parameters for each patient of the electronic database, such that the machine learning system is trained to produce a prediction, an estimation, or a likelihood of the risk for a patient to have a LTAE, in particular a torsades-de-pointes related event according to an ECG from the patient (when provided with an ECG of the patient).

In particular, the machine learning system is trained to classify the ECG in a class "at risk" or a class "not at risk", wherein the patient having an ECG classified in the "at risk" class has an increased risk of having a LTAE than a patient having an ECG classified in the "not at risk" class.

Specifically, the machine learning classifier may be a neural network, in particular a convolutional neural network, notably a DenseNet convolutional neural network. The invention thus pertains to a method for producing an artificial neural network capable of estimating the risk [providing a likelihood] for a patient to have a LTAE, in particular a torsades-de-pointes event, comprising:
a. storing, in an electronic database, a plurality of sets of data from multiple subjects comprising
   i. first parameters that are ECG from each subject,
   ii. second parameters relating to the occurrence of a LTAE, notably a torsades-de-pointes event for each subject;
b. providing a network of nodes interconnected to form an artificial neural network, the nodes comprising a plurality of artificial neurons, each artificial neuron having at least one input with an associated weight; and
c. training the artificial neural network using the plurality of first and second parameters such that the associated weight of the at least one input of each artificial neuron of the plurality of artificial neurons is adjusted in response to respective first and second parameters of the plurality of different sets of data from the multiple subjects, such that the artificial neural network is trained to produce a prediction on the risk for a patient to have a LTAE, in particular a torsades-de-pointes event, when an ECG from the patient is inputted in the neural network.

In order to properly train the machine learning classifier, it is advisable to use subjects' data (in the database) that comprises ECG from subjects in whom a LTAE, in particular a torsades-de-pointes event occurred, and ECG from patients in whom no LTAE occurred.

The ECG signal can be acquired on a single lead (preferably the V2, V5 or DII lead). It is however preferred when the ECG signal is acquired from more than one lead, preferably from at least 3, more preferably from at least 6 or from at least 8 leads. Any lead can also be used individually. DI, DII, V2, V5 or V6 leads are of particular interest.

The D2 (DII) lead is the one that usually includes the most information on ventricular repolarization, is recordable with 2 electrodes (as it's a bipolar lead that doesn't require a Wilson central terminal) and is transposable to consumer devices. It is thus the preferred lead on which the signal is acquired.

The 6 leads in the frontal plane can be calculated with the 2 leads D1 and D2. Precordial leads (V1 to V6) require 6 specific electrodes (in addition to the central terminal). They may be used, but may be less adequate, in case of a portable device that is used by the patient at his/her home, the recording being sent to a remote server as disclosed herein.

When several leads are used, one can use the information from all the leads, or "synthesizing" the information from all the leads into one "global" lead (using principal component analysis, for example). When the data has been obtained on multiple leads, the data from all the leads can be entered at once as the ECG signal (composite ECG signal, see for instance Dohare et al. (2017). Detection of Myocardial Infarction in 12 lead ECG using Support Vector Machine. Applied Soft Computing. 64. 10.1016/j.asoc.2017.12.001. I) or part of or all leads separately.

The invention also relates to a system for determining whether a patient with a congenital long QT condition has an increased risk to have a LTAE, notably torsades-de-pointes event, comprising:
a. an external device, configured to monitor electrocardiogram (ECG) data from the patient;
b. one or more processors configured to perform operations comprising:
   i. optionally performing a mathematical standardization of the ECG data for a given time domain,
   ii. using the ECG data of the patient in a machine learning classifier as disclosed above,
   iii. and determining a likelihood for the patient with a congenital long QT condition to have a torsades-de-pointes event.

The invention also relates to a subject cardiac monitoring device comprising:
a. at least one electrode for obtaining an electrocardiogram (ECG) signal from a patient;
b. a processing unit comprising at least one processor operatively coupled to the at least one electrode; and
c. at least one non-transitory computer-readable medium comprising program instructions that, when executed by the at least one processor, causes the cardiac monitoring device to:
   i. obtain the ECG signal from the at least one electrode;
   ii. optionally standardize/normalize the ECG signal to obtain a standardized/normalized ECG signal;
   iii. optionally extract data from the standardized ECG signal corresponding to a predetermined duration of time in order to obtain an extracted standardized ECG signal,
   iv. provide the ECG signal, optionally standardized/normalized and extracted, to a machine learning classifier, wherein the machine learning classifier has been obtained by a method herein described,
   thereby obtaining an output from the machine learning classifier, said output being the likelihood of having an increased risk of a TdP related event when the subject has congenital LQT condition.

The device may also be modified to acquire and process a signal that has been obtained from more than one lead.

The invention also relates to a device comprising:
a. at least 2 electrodes for obtaining an electrocardiogram (ECG) signal from a patient;
b. a processing unit comprising at least one processor operatively coupled to the at least 2 electrodes; and
c. at least one non-transitory computer-readable medium comprising program instructions that, when executed by the at least one processor, causes the cardiac monitoring device to:
   i. obtain the ECG signal from the at least one lead;
   ii. optionally standardize/normalize the ECG signal to obtain a standardized/normalized ECG signal;
   iii. optionally extract data from the standardized ECG signal corresponding to a predetermined duration of time in order to obtain an extracted standardized ECG signal,
   iv. provide the ECG signal, optionally standardized/normalized and extracted, to a machine learning classifier, wherein the machine learning classifier has been obtained by a method herein disclosed.

Consequently, the device embodies steps of methods herein disclosed (in particular when sending the ECG signal to the machine learning classifier, or standardizing/normalizing the ECG signal), and can be used to generate an output from the machine learning classifier, said output being the likelihood of having an increased risk of a LTAE, in particular TdP related event when the subject has congenital LQT condition.

The invention also relates to a device for performing the methods herein disclosed, comprising a first means to receive the ECG data of a patient, a second means for applying this ECG data to a machine learning classifier as disclosed, and a means for receiving the output from the machine learning classifier. A means for displaying the output and the information relating to the risk of having a LTAE, can also be envisaged.

Standardization of an ECG signal can be performed by any method known in the art. One can cite Kligfield et al (J Am Coll Cardiol. 2007 Mar 13;49(10):1109-27. doi: 10.1016/j.jacc.2007.01.024) and other articles from the same authors in the same journal.

As indicated above, it is also preferable to extract a segment (part) from an ECG signal (preferably during a 10-second window) in order to obtain a data segment that can be used for processing in a machine-learning system that has previously been trained as disclosed above. Such machine-learning system would thus have been trained in such segments of ECG signals. Such extraction can be done online or offline.

In particular, when the ECG is acquired at 500 Hz (500 values per second), using a 10 second window provides 5000 values, and each ECG lead is thus a vector of 5000 double values (signal and time. Standardization can be performed by
- For each EEG value of the vector, calculating the difference between such ECG value and the mean of all EEG values of the vector and
- dividing the obtained result by the standard deviation calculated for this ECG lead.

It is also possible to normalize and/or to standardize the data segment or to perform no processing at all. For instance, when the ECG signal has been acquired on multiple leads, the standardization can be performed using the mean or median of the signals acquired for each lead for the patient or per each lead. Normalization may be performed using the ECG signals of all patients that are present in a reference database or for each ECG.

The data segment is applied to the machine-learning system that has been developed as disclosed above, in order to obtain an output. Other data can also be applied together with the data segment (such as sex, age of the patient, kalemia, etc.). When the data has been obtained on multiple leads, the data from all the leads can be entered at once as the ECG signal (composite ECG signal) or part of or all leads separately.

The invention also related to a method for determining whether a patient with congenital LQT syndrome has an increased risk of having a LTAE, comprising
a) requiring a sender to identify himself within a public or private network,
b) requiring the sender to fill in information related to the patient, and assigning a specific identifier to the patient in a database, optionally after verifying that the patient has not already been assigned a specific identifier;
c) receiving data of the ECG of the patient, optionally as well as age, gender and genotype of the patient, wherein the sender is in a remote place and wherein the values are received in a secure manner, and storing the values in the database, associated with the specific identifier of the patient
d) transmitting the data to a machine learning classifier trained to provide a likelihood that the patient has an increased risk to have a LTAE, wherein the data is transferred to the machine learning classifier in a secure manner,
e) receiving the output from the machine learning classifier wherein the output is received in a secure manner,
f) storing the output in a database associated with the specific identifier, thereby obtaining a database where the information related to the patient is present under the specific identifier, and preferably the date and time of identification of the sender
g) comparing the output to a predetermined value, wherein the patient has an increased risk to have a LTAE if the end value is higher than the predetermined value, thereby obtaining a nature of the risk of occurrence of a LTAE,
h) sending the nature of the risk to the sender or to another person (such as a physician).

As for the methods described above, the risk is increased as compared to the average risk of a population of patients having the same clinical conditions (congenital LQT syndrome, preferably of the same type). This method is adapted when the recording (acquisition of the ECG signal) is performed with a consumer device at the patient's home.

In a first step, a sender is required to identify himself to a server within a public or private network. It is preferred when the network is a private network, and when the connection to the server is encrypted. Identification of the sender can be performed using login and password, preferably through strong identification processes (such as one-time password, or using biometric or smart card identification).

Upon login to the server, the sender is required to fill-in information related to the patient (such as name, sex, birth date, height, weight, social security identification number) and a specific identifier is assigned to the patient in a database. If the database doesn't contain any entry relating to the patient, one entry is created. If an entry already exists (using the social security identification number allows ensuring the unicity of entry), such entry is activated.

The server shall then receive the data from the ECG of the patient, and store them in a database, associated with the specific identifier of the patient, so that such data is uniquely and exclusively associated to the patient. Exchanges between the sender's device and the server are very advantageously performed in a secure manner (encrypted exchanges). Age and sex of the patient may also optionally be sent or may be calculated using the information related to the patient. Information relating to the genotype and of the LQT type (LQT1, LQT2 or LQT3) of the patient may also be entered or be present in the database (if previously added). Such information is also preferably received in a secure manner, and stored in the database, associated with the specific identifier of the patient.

It is to be noted that the sender is generally in a remote place different from where the server is located. It is also envisaged that the information is recovered by the server, using information provided by the sender. For instance, the server may open a connection with the server of the laboratory having performed the ECG, using identifiers provided by the sender, to upload the required information.

The next step is to transmit the ECG data to the machine learning classifier as herein disclosed, trained to provide an output that reflects the risk of the patient to have a LTAE and receive the output. Such output is stored in the database, associated with the specific identifier of the patient.

The machine learning classifier may be hosted with the server or be in a remote location. In this case, the server having the patient's information opens a (preferably encrypted) session with the server hosting the machine learning classifier system, and sends the patient's identifier and ECG data.

The output received from the machine learning classifier is then stored in a database associated with the identifier specific of the patient and is preferably date and time-stamped. Indeed, since the method may be performed on a regular basis, it may be interesting to keep a record of the results obtained overtime. Thereby, a database is obtained, containing identification related to the patient (patient's specific identifier), associated with the ECG data, and numerical values (outputs from the machine leaning classifier) that are date and time-stamped.

The output may be compared to a predetermined value, and the nature of the risk of the patient (increased risk, lower risk or average risk) of developing a LTAE is determined.

Such information (nature of the risk of the patient) is then transmitted to the sender or to another person (such as a physician).

The device may also include a storage unit / database including, for each patient, the LQTS genotype of the patient, the digital ECG signal, and the class as determined by the machine learning classifier as disclosed. A patient's identifier, patient's medications, list of contraindicated drugs, history of past events, etc., may also be included within the database.

The means for receiving the electrocardiogram (ECG) signal may comprise an analog-to-digital converting unit, which converts an analog electrocardiogram signal into a corresponding digital electrocardiogram signal. Such types of units are disclosed in particular in US8285369.

The digital ECG signal is stored within a storage unit.

The means for normalizing and/or standardizing the ECG signal comprise a storage unit and a digital signal processor. The digital ECG signal is normalized and/or standardized by the processor and the resulting data is stored in the storage unit.

The means for calculating the output (risk of having a LTAE) include a processing unit that retrieves the digital ECG signal (potentially normalized and/or standardized) and applies or sends it to machine learning classifier as disclosed above. In some embodiments, the means for calculating the output retrieves multiple ECG signals successively stored in the database, performs an average of these multiples ECG signal to obtain an average ECG signal that is stored in a database (preferably together with an associated time). In other embodiments, the means for calculating the estimated output further comprises a processing unit that calculates an average of multiple outputs and stores the results in a database.

The output may then be stored in a database (or a storing unit), preferably date and time-stamped.

The device may also comprise means for displaying the output on a monitor. It is preferred when the data stored in the storage units or the databases of the device can be exported, for further analysis, in particular by a physician.

In some embodiments, a processing unit calculates the mean, median, highest value, lowest value, standard deviation of the plurality of outputs stored in the database, when multiple ECG samples are used. Such calculated results can be stored in an appropriate database (that would also include an ID of the patient), displayed on a monitor, exported or sent to a remote terminal.

Consequently, the device specially intended for performing the methods herein disclosed makes it possible to obtain an indication about the risk for the patient to have a LTAE, according to the class (output obtained from the machine learning classifier) according to the inputted ECG.

In some embodiments, a database that is implemented during performance of the methods described above can be organized as to contain, for a heartbeat, a table containing, as data
- The digital image(s) of the ECG sample(s)
- The time (hour, minute, second) for each ECG sample
- The calculated output for each ECG sample

This table shall be in relation with another table comprising identification information for the patient (for instance name, surname identification number, birthday... ).

The invention also pertains to a computer product/program comprising program code instructions recorded on a computer-readable medium, for carrying out the steps of the methods herein disclosed, when said program is executed on a computer, as well as a computer-readable recording medium on which is recorded a computer program comprising program code instructions for performing the steps of the methods herein disclosed.

The invention also pertains to a computer program product comprising instructions to perform the methods herein disclosed, when such instructions are executed by a logical circuit or a processor.

The invention also relates to a computer-implemented method for providing a risk that for a subject with congenital Long QT syndrome to have a LTAE, comprising
a) Receiving inputs from a sender, wherein the inputs are digital ECG of the subject, and optionally information about the subject
b) Inputting the digital ECG of the subject to a machine learning classifier trained to generate likelihoods of LTAE as the output
c) Performing a method as herein disclosed, to obtain an output from the machine learning classifier
d) optionally comparing the output to a reference value and determining the risk for the patient to have a LTAE
e) Sending the output to the sender and/or the risk, if determined Optionally storing the inputs and the output in a database.

The invention also relates to a computer implemented method for obtaining information as to the risk of a subject with congenital Long QT syndrome to have a LTAE, comprising
a) Sending inputs to a remote server, wherein the inputs are digital ECG of the subject, and optionally information about the subject, wherein the remote server comprises a processor including a machine learning classifier trained to provide likelihood of occurrence of a LTAE
b) Having the processor performed a method as herein disclosed, and obtain an output
c) optionally having the remote server compare the output to a reference value and determining the risk for the patient to have a LTAE
d) Optionally having the remote server store the inputs and the output in a database
e) receiving the output from the server and/or the information pertaining to the risk for the patient to have a LTAE (when determined).

The invention also relates to a method for treating a subject, comprising performing the methods herein disclosed to evaluate whether a patient with congenital LQT syndrome has an increased risk of a LTAE, comprising performing a method herein disclosed and changing pharmacological treatment, as indicated below, or implanting an automatic implantable defibrillator (ICD) or performing left cardiac sympathetic denervation (LCSD) when an increased risk is detected.

The information pertaining to the risk of the patient can be used for refining or improving LTAE prevention in the patient, by performing other examinations if the patient is found to have an increased risk of developing a LTAE, as determined by the methods herein disclosed. One can cite changing the treatment strategy (including but not limited to hospitalization for cardiac monitoring and electrolytes dosing in case of immediate overt risk, changing drug dosage and/or prescription (*i.e*., increase betablocker, add mexiletine, ranolazine, or aldactone), according to the LQTS genotype of the patient.

In particular, if a patient is found to have "regular" risk for LTAE (i.e., a risk that is not associated with an external source), the physician can adjust the treatment, for instance increase or change the beta-blocker dosage. Beta blockers slow the heart rate and make long QT episodes less likely. One can cite nadolol and propranolol. They can also be used with mexiletine.

In contrast, when a patient appears at being at "low" risk of LTAE (ECG being classified in the "low risk" class and with outputs consistently very low (less than 0.2, more preferably less than 0.10), it might be possible to lower the amount of beta-blocker drug that is taken by the patient, which could lead to improvement of the patient's quality of life.

If a patient has a "high" risk (for instance very high outputs), implantation of an automatic implantable defibrillator (ICD) or left cardiac sympathetic denervation (LCSD) can be proposed to patients having such an increased risk as detected by the methods and devices herein disclosed.

A "transient" risk may be evidenced by the methods and devices herein disclosed, consisting in an increased risk due to an external stimulus applied to the patient, notably for a patient that is generally classified as "low risk". One can cite uptake, by the patient, of a given drug that increases the QT (such as some antibiotics, such as erythromycin, azithromycin, or some antifungal drugs, some antidepressant and antipsychotic medications or some anti-nausea medications) electrolyte imbalances (in particular caused by excessive vomiting or diarrhea, or eating disorders, such as anorexia nervosa), the physician would first act on the cause (cease the QT prolonging drug treatment, restore the electrolyte balance) in order to lower the QT and help restore classification of the patient in the "low risk" class.

### FIGURES

Figure 1: neural network architecture
Figure 2: ROC analyses and global model performance at the ECG level. ROC curve analyses for model performance in predicting cLQTS events (evnt+) in the training (upper curve) and holdout (bottom curve) sets. Left: results for model M1. Right: results for model M2.
Figure 3: ROC analyses and global model performance at the patient level. ROC curve analyses for model performance in predicting cLQTS events (evnt+) in the training (upper curve) and holdout (lower curve) sets. Left: results for model M1. Right: results for model M2.

### EXAMPLES

### Datasets

The study cohort included patients with congenital-LQT patients confirmed by genetic testing and followed over a 26-year period at the Arrhythmia unit of Bichat Hospital (Paris, France). The cohort included 487 participants with 1,083 longitudinal ECG recordings with an average of 5 ECG recordings per patient (median=3, IQR=6, min=1, max=15). The longest follow-up was for 23 years. The three most common cLQT forms were represented, with 266 cLQT1 (62% women), 188 cLQT2 (54% women), and 33 cLQT3 (45% women) patients. Median age of participants in this cohort was 28 years (IQR: 29, min=0, max=84).

213 (44%) participants in this cohort had at least one ECG recording under betablocker medication and 16 (3%) had a cardiac implantable electronic device. In this cohort, 213 participants (44%) had at least one recording performed while on betablocker medications, with 116, 88 and 9 (44%, 47% and 27%) participants for cLQT1, cLQT2, and cLQT3, respectively. ECGs were in normal sinus rhythm, except for 8 (0.7%) with supra-ventricular arrhythmia and 2 (0.2%) with either atrial and/or ventricular pacing.

In the current study 32 ECG from 19 patients were omitted as there was no information on past cardiac events. The use of these confidential, electronically processed patient data was approved by the French National Commission for Data Protection and Liberties (Commission Nationale de l'lnformatique et des Libertés).

### Model training

Two main models were trained and compared.

The first model **M1**, was trained in a set of 468 unique ECG corresponding to 468 patients (one ECG per patient).

The dataset was divided in four subsets:
- training (n=326 ECG),
- validation (n=24 ECG, validation set while trained),
- evaluation (n=47 ECG, evaluate the model for hyper parameter optimization)
   and
- holdout (n=69 ECG, assert model generalization).

31% (n=144) of the patients developed events, while 69% (n=324) did not. These proportions were similar (30%, 33%, 28%) with 75, 60 and 9 patients for each of the three cLQT groups (cLQT1, cLQT2, cLQT3). The same class labels (evnt-, evnt+) was inferred for all the ECGs of these patients, against which the model performance was compared.

The second model **M2**, was trained with more data.

The same class labels (evnt-, evnt+) was inferred for all the ECGs of these patients (n=1051) and this larger dataset was used to train the model.

The dataset was divided in four subsets:
- training (n=728 ECG),
- validation (n=48 ECG, validation set while trained),
- evaluation (n=110 ECG, evaluate the model for hyper parameter optimization) and
- holdout (n=156 ECG, assert model generalization).

31% (n=327) of the ECG were from patients developed events, while 69% (n=724) did not. These proportions were similar (29%, 33%, 39%) with 154, 147 and 26 patients for each of the three cLQT groups (cLQT1, cLQT2, cLQT3).

### Model architecture

The machine learning classifier used to predict cLQT event is based on a deep convolution neural network (CNN) trained on ECGs from patients that are labelled has having had an event. As indicated above, it is known that such patients have an increased risk to have a new LTAE, and the inventors postulated that such risk could be "seen" in their EEGECG, the EEGECG containing some electrophysiological "markers" of the risk. The network is mainly composed of three types of layers, i.e., *convolutional, pooling* and *fully connected layers.* Below is an overall description of the model, followed by a specific implementation of hyperparameters. *Convolutional layers* generate matrix transformations of input data by computing different filter-based *convolution* operations. Such layers where hundreds to thousands of different filters are applied, aim to automatically extract abstract features from the data.

The *pooling layers* down-sample extracted features through max, min or average operations to produce a more condensed resolution, thus allowing spatial dimensionality reduction of the data. Although, information is lost, this remains necessary to improve computational efficiency and to limit overfitting.

In the *fully connected layers,* each node in the output layer is directly fed from all nodes in the previous layers.

Altogether, these types of layers are used to build a more complex architecture based on the DenseNet approach. Unlike traditional linear convolutional architectures which consist of stacking convolutions and down-sampling layers, DenseNet extracts ECG features and reuses them later in the network. Therefore, each layer receives inputs from all the preceding ones and passes its own output to all subsequent layers. The consequence is that the final output layer has direct information from every layer since the input, which optimizes computation, while allowing complex multi-level representation of the features.

The neural network architecture and is described as follows:
- It begins with a *convolutional layer* followed by a batch-normalization and Leaky ReLU activation. During this first step, higher features representations are extracted.
- The next step consists of eight successive densely connected convolutional blocks *(DenseBlocks).* A DenseBlock is made of several convolutional subblocks called *(DenseLayers).*
- Each DenseLayer begins with four layers of bottleneck composed of a batch-normalization, followed by a Leaky ReLU activation, a 1x1 convolution and a dropout layer. This bottleneck reduces the filter space dimensionality, decreasing the number of feature maps whilst retaining their salient features. The bottleneck technique helps reduce the number of parameters and increases computational efficiency. After the bottleneck comes another batch-normalization layer, a Leaky ReLU activation, a classic convolution, and another dropout layer. The dropout rate is a hyper-parameter and is determined before training. DenseLayers are densely connected, meaning that each DenseLayer takes as input all previous DenseLayers outputs.
- Between each DenseBlock lies a transition block with a bottleneck, followed by a Leaky ReLU activation and an average pooling 1D layer which takes the average of points, reducing the dimensionality of the previous output. The transition block aims to down sample the data flow through the network and interconnect the DenseBlocks.
- The signal is progressively down sampled, then we compute the average the output of the last convolution through an *GlobalAveragePooling* layer
- After convolutions layers, five stacked non-CNN fully connected layers are responsible for the classification, the last layer has two neurons which outputs are computed through a SoftMax activation function to get classification scores for each class. The score of each class ranges from 0 to 1.

The structure of the neural network architecture is depicted on Figure 1.

To obtain best performances, a hyper-optimization process was run using grid search algorithm. Several models with different combinations of parameters are tested and evaluated. The best combination giving the model with highest performances is given in Table 1: Model hyper-parameters.

**Table 1: Model hyper-parameters**

| **Training Process Parameters** | |
|---|---|
| *Weights optimizer algorithm* | Adam |
| *Optimizer learning rate* | 0.0001 |
| *Loss function* | Sigmoid binary cross entropy |
| *Number of iteration*/*epochs* | 400 |
| *Batch size* | 512 samples per GPUs, we used 4 GPUs simultaneously for a total batch size of 2048 |

| **Model Specific Parameters** | |
|---|---|
| *Dense Blocks* | 6 |
| *Dense Layers* | 4 |
| *Bottleneck* | Before |
| *Compression* | 1.0 |
| *Dropout rate* | 0.2 |
| *Initial weights distribution* | Xavier distribution |

### Results

### Model performance

Only lead II was used from the 12 lead, 10 seconds ECG in this study. The data are XML files containing anonymized ECG signal acquired at 500Hz. Each ECG lead is thus a vector of 5000 double values (signal and time). The DII lead is known to allow good recognition of waves represents a QRS complex with positive polarity. Standardization of this signal is performed prior to training. Indeed, ECG amplitude varies widely. These large variations may disrupt the model, alter learning and prevent convergence. Standardizing the ECG signals limits the variations, so that the learns better and converges faster and thus necessitates less ECG during the learning phase.

For each individual ECG, standardization is performed by
- Calculating the difference between each ECG value of the vector and the mean of all EEG values of the vector and
- dividing the calculated difference by the standard deviation of the EEG values of the vector.

Normalisation usually places the ECG in a range between 0 and 1, which can have the effect of crushing the signal. It was thus not performed in this example.

No other transformation was performed. The output of the CNN models consists of two scores ranging from [0,1] for the EVNT+ and EVNT- class. Only the first score is used here. The EVNT+ class corresponds to the signal belonging to patients having had a LTAE prior to the learning, and the EVNT- class corresponds to the signal of the patients without LTAE. As indicated above, it is postulated that prior LTAE increases the risk of future LTAE.

An ECG is classified as EVNT+ if the score is >0.5.

Both M1 and M2 models were trained in the training set and tested in the holdout set, which was never used for training, nor for hyperparameter optimization. The datasets were split at the patient level to avoid intra-patient contamination. Performance was computed at the ECG level, for each ECG specifically but also at the patient level, using a voting approach (i.e., the risk scores predicted for each ECG were averaged for ECGs from each patient).

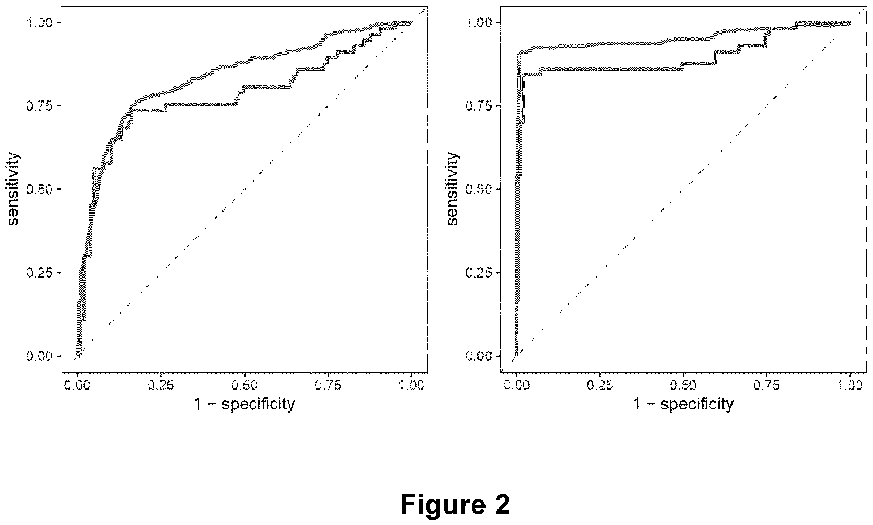

The overall training ROC AUC for M1 was 0.84 CI=[0.8; 0.87] and for the holdout set it was 0.78 CI=[0.7; 0.87]. The overall training ROC AUC for M2 was 0.95 CI=[0.93; 0.98] and for the holdout set it was 0.9 CI=[0.84; 0.96] (**Figure 2**). The higher performance of M2 is likely explained by the greater number of ECG (2.24 times more ECG).

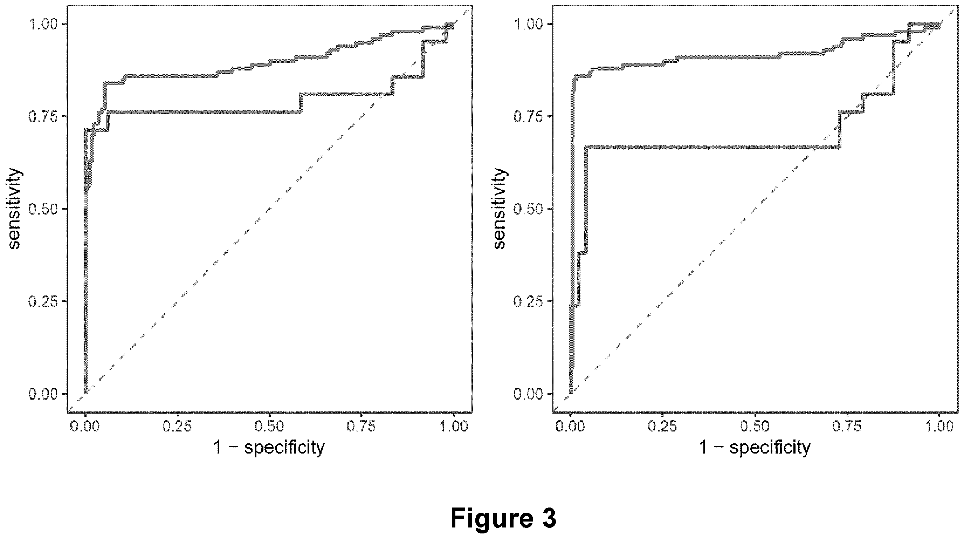

When tested at the patient level with the voting approach, the overall training ROC AUC for M1 was 0.9 CI=[0.85; 0.94] and for the holdout set it was 0.8 CI=[0.63; 0.96]. The overall training ROC AUC for M2 was 0.92 CI=[0.87; 0.96] and for the holdout set it was 0.71 CI=[0.54; 0.88] (**Figure 3**). Although results improved for M1, they did not for M2 and the overall confidence interval widened for both models. This indicates that although markers visible by the models exist in all ECG they can vary through time.

Next, the performance analyses were performed for each cLQTS type, to explore for potential specificities.

In cLQT1, at the ECG level, the training ROC AUC for M1 was 0.81 CI=[0.76; 0.86] and for the holdout set it was 0.77 CI=[0.6; 0.94]. For M2, the ROC AUC was 0.95 CI=[0.92; 0.98] and for the holdout set it was 0.77 CI=[0.6; 0.94]. There seems to be some overfitting here. Similar results were observed at the Patient level. The training ROC AUC for M1 was 0.87 CI=[0.79; 0.95] and for the holdout set it was 0.77 CI=[0.53; 1]. For M2, the ROC AUC was 0.91 CI=[0.84; 0.98] and for the holdout set it was 0.59 Cl=[0.31; 0.86]. The overfitting was even more important in this case with very large confidence intervals.

In cLQT2, at the ECG level, the training ROC AUC for M1 was 0.89 CI=[0.85; 0.94] and for the holdout set it was 0.77 CI=[0.67; 0.88]. For M2, the ROC AUC was 0.96 CI=[0.92; 0.99] and for the holdout set it was 0.95 CI=[0.9; 1]. The performance is very high here with no overfitting. Similar results were observed at the Patient level but with more overfitting as seen previously. The training ROC AUC for M1 was 0.96 CI=[0.92; 1] and for the holdout set it was 0.8 CI=[0.54; 1]. For M2, the ROC AUC was 0.95 CI=[0.9; 1] and for the holdout set it was 0.84 CI=[0.63; 1].

In cLQT3, at the ECG level, the training ROC AUC for M1 was 0.73 CI=[0.58; 0.89] and for the holdout set it could not be computed due to very few observations left. For M2, the ROC AUC was 0.94 CI=[0.85; 0.1] and for the holdout set it could not be computed due to very few observations. Similar results were observed at the Patient level. The training ROC AUC for M1 was 0.62 CI=[0.27; 0.98] and for the holdout set it could not be computed due to very few observations. For M2, the ROC AUC was 0.71 CI=[0.34; 1] and for the holdout set it could not be computed due to very few observations. The overfitting was even more important in this case with very large confidence intervals.

### Previous TdP risk model performance on the EVNT+ / EVNT- classification

The analyses of the previous models described in WO2020245322, respectively the multilead model and the unilead Dll model which were trained to quantify the risk for torsades-de-pointes events. These models were tested in discriminating the EVNT+/EVNT- classes described above. Both models did not perform well in this task.

This shows that a model developed on a general population, using a inhibitor of iKr channel is not adapted for determining the occurrence of TdP for patients with congenital LQT syndrome. This result is surprising, as the previous model was able to identify patients with LQT2 syndrome.

## Claims

1. A method for determining whether a patient with a congenital long QT syndrome has an increased risk of having a torsades-de-pointes related event (i.e., syncope, aborted cardiac arrest, sudden cardiac death), comprising:
a. receiving, by a processing device, signal data representing a time segment of an ECG waveform of a subject patient
b. analyzing, by the processing device *via* a configured artificial machine learning classifier trained to provide likelihoods of occurrence of a torsades-de-pointes related event, as the output of the machine learning classifier,
wherein a likelihood higher than 0.5 indicates an increased risk for the patient to have a torsades-de-pointes related event.

2. A method for detecting whether a given patient with a congenital long QT condition has an increased risk to present an episode of torsades-de-pointes, comprising the steps of:
a. Obtaining ECG data from the patient;
b. applying the ECG data to a machine learning classifier configured to detect patterns in the ECG data indicative of a risk for the patient to have a torsades-de-pointes event,
c. obtaining an output from the machine learning classifier, wherein the output provides a likelihood of the risk for the patient to have a torsades-de-pointes event.

3. The method of claim 1 or 2, wherein the signal data or the ECG data is sent to a remote machine learning classifier and wherein the output is sent to the patient and/or to a physician.

4. The method of any one of claims 1 to 3, wherein the patient is assigned to a class of risk of having a torsades-de-pointes event by
a. repeating the methods with different ECG signals from the same patient and
b. assigning the patient in the class (increased risk / no increased risk) for which the majority of the outputs or the mean of the ouputs is assigned.

5. The method of claim 4, wherein the different ECG signals are signals acquired during a period of time of at least 12 hours, and wherein the patient has an increased risk of having a torsades-de-pointes event if the majority of the outputs obtained for each ECG signal is in the "increased risk" class.

6. The method of claim 4, wherein the different ECG signals are signals acquired during a period of time of at least 12 hours, and wherein the patient has an increased risk of having a torsades-de-pointes event if at least one ECG signal is the "increased risk" class above a predetermined threshold value.

7. A method for producing a machine learning classifier capable of estimating the risk for a patient with congenital Long QT syndrome to have a torsades-de-pointes related event, comprising:
a. storing in an electronic database patient data comprising
i. a first parameter that is ECG from various patient,
ii. a second parameter relating to the occurrence of a torsades-de-pointes event (or the non-occurrence) for each patient;
b. providing a machine learning classifier; and
c. training the machine learning classifier using the patient's data, such that the machine learning classifier is trained to produce a likelihood of the risk for a patient to have a torsades-de-pointes related event when an ECG from the patient is provided.

8. The method of any one of claims 1 to 7, wherein the machine learning classifier is a Convolutional Neural Network, in particular a DenseNet Convolutional Neural Network.

9. A method for producing an artificial neural network capable of providing a likelihood of the risk for a patient with congenital Long QT syndrome to have a torsades-de-pointes event during, comprising:
a. storing in an electronic database data for multiple subjects with congenital Long QT syndrome comprising
i. a first parameter that is ECG from each subject,
ii. a second parameter relating to the occurrence of a torsades-de-pointes event during the for the subject;
b. providing a network of nodes interconnected to form an artificial neural network, the nodes comprising a plurality of artificial neurons, each artificial neuron having at least one input with an associated weight; and
c. training the artificial neural network using the subjects data such that the associated weight of the at least one input of each artificial neuron of the plurality of artificial neurons is adjusted in response to respective first and second parameters of a plurality of different sets of data from the subjects data, such that the artificial neural network is trained to produce a prediction on the risk for a patient to have a torsades-de-pointes event and cause thereof when exposed to an ECG from the patient.

10. The method of any one of claims 7 to 9, wherein the subjects data comprises ECG from subjects with congenital Long QT syndrome in whom a torsades-de-pointes event occurred, and ECG from subjects with congenital Long QT syndrome in whom a torsades-de-pointes event didn't occur.

11. The method of any one of claims 1 to 10, wherein the ECG signal is an ECG signal from at least one lead.

12. The method of any one of claims 1 to 10, wherein the ECG signal has been standardized to reduce the amplitude of the native signal.

13. A computer-implemented method for providing a risk that for a subject with congenital Long QT syndrome to have a LTAE, comprising
a) Receiving inputs from a sender, wherein the inputs are digital ECG of the subject, and optionally information about the subject
b) Inputting the digital ECG of the subject to a machine learning classifier trained to generate likelihoods of LTAE as the output
c) Performing a method of any one of claims 1 to 6 and 11 to 13, to obtain an output from the machine learning classifier
d) optionally comparing the output to a reference value and determining the risk for the patient to have a LTAE
e) Sending the output to the sender and/or the risk, if determined Optionally storing the inputs and the output in a database.

14. A computer implemented method for obtaining information as to the risk of a subject with congenital Long QT syndrome to have a LTAE, comprising
a) Sending inputs to a remote server, wherein the inputs are digital ECG of the subject, and optionally information about the subject, wherein the remote server comprises a processor including a machine learning classifier trained to provide likelihood of occurrence of a LTAE
b) Having the processor performed the method of any one of claims 1 to 6 and 11 to 13, and obtain an output
c) optionally having the remote server compare the output to a reference value and determining the risk for the patient to have a LTAE
d) Optionally having the remote server store the inputs and the output in a database
e) receiving the output from the server and/or the information pertaining to the risk for the patient to have a LTAE (when determined).

15. A system for determining whether a patient with a congenital long QT condition has an increased risk to have a torsades-de-pointes event, comprising:
a. an external device, configured to monitor electrocardiogram (ECG) data from the patient;
b. one or more processors configured to perform operations comprising:
i. optionally performing a mathematical standardization of the ECG data for a given time domain,
ii. performing a calculation by a machine learning classifier
iii. and determining a likelihood for the patient with a congenital long QT condition to have a torsades-de-pointes event.
